# EUROPÄISCHE PATENTSCHRIFT

(11) **EP 1 527 060 B1**
(45) Veröffentlichungstag und Bekanntmachung des Hinweises auf die Patenterteilung: **09.08.2006**
(21) Anmeldenummer: 03765084.3
(22) Anmeldetag: 22.07.2003
(51) Int. Cl.: C07D 301/32, B01D 3/32

(54) **VERFAHREN ZUR KONTINUIERLICH BETRIEBENEN REINDESTILLATION DES BEI DER KOPPELPRODUKTFREIEN SYNTHESE VON PROPYLENOXID ANFALLENDEN 1,2-PROPYLENGLYKOLS**
METHOD FOR THE CONTINUOUS PURIFICATION BY DISTILLATION OF 1,2-PROPYLENE GLYCOL THAT ACCUMULATES DURING THE SYNTHESIS OF PROPYLENE OXIDE WITHOUT COUPLING PRODUCTS
PROCEDE DE PURIFICATION PAR DISTILLATION EN CONTINU DU 1,2-PROPYLENE GLYCOL PRODUIT AU COURS DE LA SYNTHESE D'OXYDE DE PROPYLENE SANS CO-PRODUITS

(30) Priorität: 23.07.2002 DE 10233382
(43) Veröffentlichungstag der Anmeldung: 04.05.2005
(73) Patentinhaber: BASF Aktiengesellschaft, 67056 Ludwigshafen (DE)
(72) Erfinder: BASSLER, Peter, 68519 Viernheim (DE); GÖBBEL, Hans-Georg, 67169 Kallstadt (DE); TELES, Joaquim, Henrique, 67166 Otterstadt (DE); RUDOLF, Peter, 68526 Ladenburg (DE)
(74) Vertreter: Hörschler, Wolfram Johannes
(86) Internationale Anmeldenummer: PCT/EP2003/007985
(87) Internationale Veröffentlichungsnummer: WO 2004/009571

(56) Entgegenhaltungen:
- US-A- 3 574 772
- KAIBEL G: "DISTILLATION COLUMNS WITH VERTICAL PARTITIONS" CHEMICAL ENGINEERING AND TECHNOLOGY, WEINHEIM, DE, Bd. 10, 1987, Seiten 92-98, XP002939161 ISSN: 0930-7516

## Beschreibung

Die Erfindung betrifft ein kontinuierlich betriebenes Verfahren zur Reindestillation des bei der vorzugsweise koppelproduktfreien Synthese von Propylenoxid als Nebenprodukt anfallenden 1,2-Propylenglykols, wobei das bei der Synthese entstehende Gemisch, das das 1,2-Propylenglykol enthält, in einer Trennwandkolonne in eine Leicht-, Mittel- und Hochsiederfraktion aufgetrennt und das 1,2-Propylenglykol als Mittelsieder aus dem Seitenabzug der Kolonne entnommen wird. Die US 3 574 772 beschreibt ein verfahren zur Herstellung von 1,2-Propylenglykol aus Propylenoxid, worin das rohe 1,2-Propylenglykol destillativ am Seitenabzug einer kolonne erhalten wird.

Nach den gängigen Verfahren des Standes der Technik kann Propylenoxid durch Umsetzung von Propylen mit Hydroperoxiden hergestellt werden, wobei diese Umsetzungen einstufig oder mehrstufig durchgeführt werden können.

Beispielsweise kann das in der WO 00/07965 beschriebene mehrstufige Verfahren zur Herstellung von Propylenoxid durch Umsetzung von Propylen mit einem Hydroperoxid durch ein Reaktionsschema beschrieben werden, das wenigstens die Schritte (i) bis (üi) umfasst:
(i) Umsetzung des Hydroperoxids mit Propylen unter Erhalt eines Produktgemisches, umfassend Propylenoxid und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit Propylen.

Demgemäss findet die Umsetzung von Propylen mit dem Hydroperoxid in mindestens zwei Stufen (i) und (iii) statt, wobei das in Stufe (ii) abgetrennte Hydroperoxid erneut in die Reaktion eingesetzt wird.

Vorzugsweise erfolgt dabei die Umsetzungen in den Stufen (i) und (iii) in zwei getrennten Reaktoren, vorzugsweise Festbettreaktoren, wobei vorzugsweise die Umsetzung der Stufe (i) in einem isothermen und die Umsetzung der Stufe (iii) in einem adiabatischen Reaktor stattfindet.

Vorzugsweise wird bei dieser Sequenz als Hydroperoxid Wasserstoffperoxid verwendet und Propylen während der Reaktion in Kontakt mit einem heterogenen Katalysator gebracht, wobei die Umsetzung in Methanol als Lösungsmittel durchgeführt wird.

Hierbei erreicht der Wasserstoffperoxid-Umsatz in Stufe (i) ca. 85 % bis 90 % und in Stufe (iii) ca. 95 % bezogen auf Stufe (ii). In der Summe kann über beide Stufen ein Wasserstoffperoxidumsatz von ca. 99 % bei einer Propylenoxid-Selektivität von ca. 94 - 95 % erreicht werden.

Wegen der hohen Selektivität der Reaktion wird diese Synthese auch als koppelproduktfreie Propylenoxidsynthese bezeichnet.

Als Nebenreaktionen der Propylenoxid-Bildung treten die Folgereaktionen des Propylenoxids mit Methanol, Wasser und Wasserstoffperoxid auf, wobei im Vergleich zum Propylenoxid höhersiedende Produkte entstehen. Als Reaktionsnebenprodukte bilden sich durch Addition von Methanol an Propylenoxid die Methoxypropanole, durch Addition von Wasser 1,2-Propylenglykol, Dipropylenglykol und Tripropylenglykol, und durch Addition von Wasserstoffperoxid 2-Hydroperoxy-1-propanol und 1-Hydroperoxy-2-propanol. Es ist bevorzugt, die Hydroperoxyalkohole zu reduzieren, wobei gleichfalls 1,2-Propylenglykol gebildet wird. Beispielsweise können zur Reduktion die in der DE 101 05 527.7 beschriebenen Methoden verwendet werden.

Nach Abtrennung und Rückführung des Lösungsmittels Methanol verbleiben die Reaktionsnebenprodukte im Abwasser und können daraus als Nebenausbeute isoliert werden.

Insbesondere ist im Abwasser mit 1,2-Propylenglykol ein Wertstoff vorhanden, der vielfältige Anwendungen findet. Beispielsweise kann er zur Herstellung von Polyetheralkoholen, als Veresterungskomponente in Polyestersynthesen, zur Herstellung von Polyurethanen, als reaktives Lösungsmittel für Polyurethansynthesen mit der Anwendung in Beschichtungen und Lacken oder als Filmbildungshilfsmittel verwendet werden. Hochreines Propylenglykol wird ansonsten in der technischen Fertigung durch Umsetzung von Propylenoxid mit Wasser bei Drücken zwischen 15 und 25 bar und einer Temperatur von 180 bis 220 °C hergestellt. Die Aufarbeitung ist aufwendig, da zur Abtrennung des Propylenglykols drei in Reihe geschaltete Kolonnen eingesetzt werden müssen (Ullmann's Encyclopädie der technischen Chemie, Verlag Chemie, 4. Auflage, Seite 425 bis 431). Weiterhin sind zur Propylenglykolherstellung aber auch alle möglichen Verfahren zur katalytischen Addition von Wasser an Propylenoxid anwendbar, beispielsweise die in der WO 99/31034 beschriebene Methode.

Zur Abtrennung des Propylenglykols aus dem bei der Propylenoxidherstellung anfallenden Abwasser werden nach den bisherigen Abtrennverfahren Destillationskolonnen mit Seitenabzug oder in Reihe geschaltete Kolonnen eingesetzt. Auch diese Vorgehensweise erfordert jedoch einen relativ hohen apparativen und energetischen Aufwand, der unwirtschaftlich ist. Häufig wird deshalb auf die Isolierung des Wertstoffes verzichtet und das den Wertstoff enthaltende Abwasser einer Verbrennung zugeführt.

Aufgabe der vorliegenden Erfindung war es, die destillative Abtrennung des bei der Umsetzung von Propylen mit Hydroperoxiden zu Propylenoxid als Nebenprodukt anfallenden und im Abwasserstrom enthaltenen 1,2-Propylenglykols unter Absenkung des sonst üblichen Energiebedarfs zu optimieren.

Diese Aufgabe konnte durch ein kontinuierlich betriebenes Verfahren zur Reindestillation des bei der vorzugsweise koppelproduktfreien Synthese von Propylenoxid als Nebenprodukt anfallenden und im Abwasserstrom enthaltenen 1,2-Propylenglykols mittels einer Trennwandkolonne gelöst werden.

Gegenstand der Erfindung ist somit ein kontinuierlich betriebenes Verfahren zur Reindestillation des bei der Synthese von Propylenoxid als Nebenprodukt anfallenden 1,2-Propylenglykols, dadurch gekennzeichnet, dass das bei der Synthese entstehende Gemisch, das das 1,2-Propylenglykol enthält, in einer Trennwandkolonne in eine Leicht-, Mittel- und Hochsiederfraktion aufgetrennt und am Seitenabzug der Kolonne 1,2-Propylenglykol als Mittelsieder entnommen wird.

Nach dem erfindungsgemäßen Destillationsverfahren kann das 1,2-Propylenglykol durch destillative Abtrennung in hoher Reinheit direkt aus dem Abwasserstrom isoliert werden, der bei der Propylensynthese anfällt. Im Vergleich zu den beim Stand der Technik geschilderten Destillationsverfahren führt das neue erfindungsgemäße Verfahren zu einem reduzierten apparativen und energetischen Aufwand bei gleichzeitig verbesserter Produktqualität. Darüber hinaus zeichnet sich die Trennwandkolonne durch einen besonders niedrigen Energieverbrauch aus und bietet somit hinsichtlich des Energiebedarfs gegenüber einer konventionellen Kolonne Vorteile.

Gleichzeitig wird durch das neue Verfahren eine hohe Wertsteigerung erzielt, da das bei der Propylenoxid-Synthese als Nebenprodukt anfallende und im Abwasserstrom enthaltene 1,2-Propylenglykol wirtschaftlich isoliert werden kann. Für die industrielle Anwendung ist das neue Verfahren außerordentlich vorteilhaft.

Destillationskolonnen mit Seitenabzügen und Trennwand, im Folgenden auch als Trennwandkolonnen bezeichnet, sind bereits bekannt. Sie stellen eine Weiterentwicklung von Destillationskolonnen dar, die nur über einen Seitenabzug, jedoch über keine Trennwand verfügen. Die Anwendungsmöglichkeit des zuletzt genannten Kolonnentyps ist aber eingeschränkt, weil die an den Seitenabzugsstellen entnommenen Produkte nie völlig rein sind. Bei Seitenabnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Bei Seitenabnahmen im Abtriebsteil der Kolonne, die meist dampfförmig erfolgen, weist das Seitenprodukt noch Hochsiederanteile auf. Die Verwendung von konventionellen Seitenabzugskolonnen ist daher auf Fälle begrenzt, in denen verunreinigte Seitenprodukte zulässig sind.

Beim Einbau einer Trennwand in eine solche Kolonne kann jedoch die Trennwirkung verbessert werden. Bei dieser Bauart ist es möglich, Seitenprodukte in reiner Form zu entnehmen. Im mittleren Bereich oberhalb und unterhalb der Zulaufstelle und der Seitenentnahmestelle ist eine Trennwand angebracht, wobei diese fest verschweißt oder auch nur gesteckt werden kann. Sie dichtet den Entnahmeteil gegenüber dem Zulaufteil ab und unterbindet in diesem Kolonnenteil eine Quervermischung von Flüssigkeits- und Brüdenströmen über den gesamten Kolonnenquerschnitt. Hierdurch verringert sich bei der Auftrennung von Vielstoffgemischen, deren Komponenten ähnliche Siedepunkte besitzen, die Zahl der insgesamt benötigten Destillationskolonnen.

Dieser Kolonnentyp wurde beispielsweise zur Trennung einer Komponentenvorlage aus Methan, Ethan, Propan und Butan verwendet (US 2,471,134), zur Trennung eines Gemisches von Benzol, Toluol und Xylol (US 4,230,533) sowie zur Trennung eines Gemisches aus n-Hexan, n-Heptan und n-Octan (EP 0 122 367).

Auch zur Trennung azeotrop siedender Mischungen können Trennwandkolonnen erfolgreich eingesetzt werden (EP 0 133 510).
Schließlich sind Trennwandkolonnen bekannt, in denen chemische Reaktionen unter gleichzeitiger Destillation der Produkte durchgeführt werden können. Als Beispiele werden Veresterungen, Umesterungen, Verseifungen sowie Acetalisierungen genannt (EP 0 126 288).

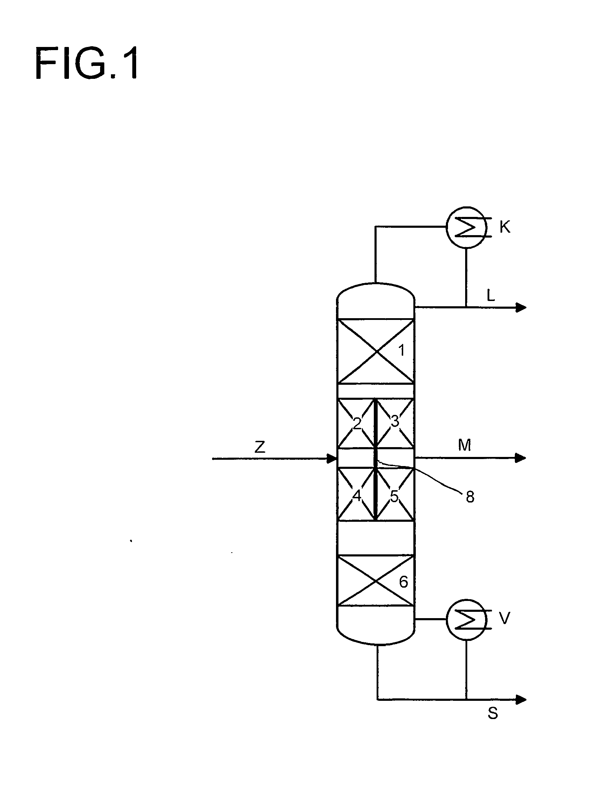

In Figur 1 ist schematisch die Abtrennung des bei der Propylenoxid-Synthese als Nebenprodukt entstehenden und im Abwasserstrom enthaltenen 1,2-Propylen-glykols in einer Trennwandkolonne dargestellt. Dabei wird der aus der Propylenoxidsynthese stammende Abwasserstrom über den Zulauf Z in die Kolonne eingebracht. In der Kolonne wird besagter Abwasserstrom aufgetrennt in eine Fraktion, enthaltend die Leichtsieder L und bestehend im wesentlichen aus Wasser und Methoxypropanolen, und in eine Fraktion, die die Hochsieder S enthält, bestehend unter anderem aus Di- und Tripropylenglykol. Aus dem Seitenabzug für Mittelsieder M kann das 1,2-Propylenglykol entnommen werden.

Zur Entnahme an der Seitenentnahmestelle eignen sich sowohl innenliegende als auch außerhalb der Kolonne angeordnete Auffangräume, in denen die Flüssigkeit oder kondensierender Dampf gesammelt werden kann.

Zur Durchführung des erfindungsgemäßen Verfahrens können übliche Trennwandkolonnen, wie sie etwa beim Stand der Technik genannt sind, verwendet werden.

Eine solche Trennwandkolonne besitzt beispielsweise vorzugsweise 15 bis 60, besonders bevorzugt 25 bis 50, theoretische Trennstufen. Mit dieser Ausführung kann das erfindungsgemäße Verfahren besonders günstig durchgeführt werden.

Dabei weist der obere gemeinsame Teilbereich 1 des Zulauf- und Entnahmeteils der Trennwandkolonne vorzugsweise 5 bis 50 %, besonders bevorzugt 15 bis 30 %, der Verstärkungsteil 2 des Zulaufteils vorzugsweise 5 bis 50 %, besonders bevorzugt 15 bis 30 %, der Abtriebsteil 4 des Zulaufteils vorzugsweise 5 bis 50 %, besonders bevorzugt 15 bis 30 %, der Abtriebsteil 3 des Entnahmeteils vorzugsweise 5 bis 50 %, besonders bevorzugt 15 bis 30 %, der Verstärkungsteil 5 des Entnahmeteils vorzugsweise 5 bis 50 %, besonders bevorzugt 15 bis 30 %, und der gemeinsame untere Teilbereich 6 des Zulauf- und Entnahmeteils der Kolonne vorzugsweise 5 bis 50 %, besonders bevorzugt 15 bis 30 %, der Gesamtzahl der theoretischen Trennstufen der Kolonne auf.

Vorzugsweise beträgt die Summe der Zahl der theoretischen Trennstufen der Teilbereiche 2 und 4 im Zulaufteil 80 bis 110 %, besonders bevorzugt 90 bis 100 %, der Summe der Zahl der Trennstufen der Teilbereiche 3 und 5 im Entnahmeteil.

Gleichfalls günstig ist es, die Zulaufstelle und die Seitenabzugsstelle hinsichtlich der Lage der theoretischen Trennstufen auf unterschiedlicher Höhe in der Kolonne anzuordnen. Vorzugsweise ist die Zulaufstelle um 1 bis 8, besonders bevorzugt um 3 bis 5, theoretische Trennstufen höher oder niedriger angeordnet als die Seitenabzugsstelle.

Die beim erfindungsgemäßen Verfahren verwendete Trennwandkolonne kann vorzugsweise sowohl als Packungskolonne mit Füllkörpern oder geordneten Packungen als auch Bodenkolonne ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Vorzugsweise wird bei vorstehend genannter Ausführung der Kolonne der durch die Trennwand 7 unterteilte Teilbereich der Kolonne, bestehend aus dem Verstärkungsteil 2 des Zulaufteils, dem Abtriebsteil 3 des Entnahmeteils, dem Abtriebsteil 4 des Zulaufteils und dem Verstärkungsteil 5 oder Teilen davon, mit geordneten Packungen oder Füllkörpern bestückt und die Trennwand in diesen Teilbereichen wärmeisolierend ausgeführt.
Der Abwasserstrom, der die hochsiedenden Nebenprodukte mit dem 1,2-Propylenglykol enthält, wird nun über den Zulauf Z in die Kolonne eingebracht. Dieser Zulaufstrom ist im Allgemeinen flüssig. Es kann jedoch von Vorteil sein, besagten Zulaufstrom einer Vorverdampfung zu unterziehen, und anschließend zweiphasig, d. h. gasförmig und flüssig oder in Form eines gasförmigen und eines flüssigen Stromes der Kolonne zuzuführen. Die Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Zweckmäßigerweise wird der Zulaufstrom mittels einer Pumpe oder über eine statische Zulaufhöhe von mindestens 1 m mengengeregelt in den Zulaufteil der Trennwandkolonne aufgegeben. Vorzugsweise erfolgt diese Zugabe über eine Kaskadenregelung in Verbindung mit der Flüssigkeitsstandregelung des Auffangraumes des Zulaufteils. Dabei wird die Regelung so eingestellt, dass die auf das Verstärkungsteil 2 aufgegebene Flüssigkeitsmenge nicht unter 30 % des Normalwertes sinken kann. Es hat sich gezeigt, dass eine derartige Vorgehensweise zur Kompensation von störenden Schwankungen bezüglich der Zulaufmenge oder der Zulaufkonzentration wichtig ist.

Ähnlich wichtig ist, dass die Aufteilung der aus dem Abtriebsteil 3 des Entnahmeteils der Kolonne ablaufenden Flüssigkeit auf den Seitenabzug für Mittelsieder M und auf den Verstärkungsteil 5 des Entnahmeteils durch eine Regelung so eingestellt wird, dass die auf den Teilbereich 5 aufgegebene Flüssigkeitsmenge nicht unter 30 % des Normalwertes sinken kann.

Die Einhaltung dieser Voraussetzungen muss durch entsprechende Regelvorschriften sichergestellt werden.

Regelungsmechanismen zum Betreiben von Trennwandkolonnen wurden beispielsweise beschrieben in Chem. Eng. Technol. 10 (1987) 92-98, Chem.-Ing.-Technol. 61 (1989) Nr. 1, 16-25, Gas Separation and Purification 4 (1990) 109-114, Process Engineering 2 (1993) 33-34, Trans IChemE 72 (1994) Part A 639-644, Chemical Engineering 7 (1997) 72-76. Die bei diesem Stand der Technik angegebenen Regelungsmechanismen können auch für das erfindungsgemäße Verfahren angewendet bzw. auf dieses übertragen werden.

Für die kontinuierlich betriebene. Reindestillation des 1,2-P.ropylenglykols hat sich nachfolgend beschriebenes Regelungsprinzip als besonders günstig erwiesen. Es ist in der Lage, Lastschwankungen gut zu verkraften. Vorzugsweise erfolgt somit die Destillatentnahme temperaturgeregelt.

Im gemeinsamen Teilbereich 1 des Zulauf- und Entnahmeteils ist eine Temperaturregelung vorgesehen, die als Stellgröße die Ablaufmenge, das Rücklaufverhältnis oder bevorzugt die Rücklaufmenge nutzt. Dabei befindet sich die Messstelle für die Temperaturregelung vorzugsweise um 3 bis 8, besonders bevorzugt um 4 bis 6, theoretische Trennstufen unterhalb des oberen Endes der Kolonne.

Durch eine geeignete Temperatureinstellung wird dann die aus dem Teilbereich 1 ablaufende Flüssigkeit am oberen Ende der Trennwand so aufgeteilt, dass das Verhältnis des Flüssigkeitsstroms zum Zulaufteil zu dem zum Entnahmeteil vorzugsweise 0,1 bis 1,0, besonders bevorzugt 0,3 bis 0,6, beträgt.

Vorzugsweise wird bei dieser Methode die ablaufende Flüssigkeit in einem in oder außerhalb der Kolonne angeordneten Auffangraum gesammelt, woraus sie dann kontinuierlich in die Kolonne eingespeist wird. Dieser Auffangraum kann somit die Aufgabe einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglicht. Bei der Verwendung von gepackten Kolonnen wird die Flüssigkeit zunächst in Sammler gefasst und von dort aus in einen innenliegenden oder außenliegenden Auffangraum geleitet.

Der Brüdenstrom am unteren Ende der Trennwand 7 wird durch die Wahl und/oder Dimensionierung der Trenneinbauten und/oder den Einbau druckmindernder Vorrichtungen, beispielsweise von Blenden, so eingestellt, dass das Verhältnis des Brüdenstroms im Zulaufteil zu dem des Entnahmeteils vorzugsweise 0,8 bis 1,2, besonders bevorzugt 0,9 bis 1,1, beträgt.

Beim vorstehend genannten Regelprinzip ist des weiteren eine Temperaturregelung im unteren gemeinsamen Teilbereich des Zulauf- und Entnahmeteils 6 vorgesehen, die als Stellgröße die Sumpfentnahmemenge nutzt. Somit kann die Entnahme des Sumpfprodukts temperaturgeregelt erfolgen. Dabei ist die Messstelle für die Temperaturregelung vorzugsweise um 3 bis 6, besonders bevorzugt 4 bis 6, theoretische Trennstufen oberhalb des unteren Endes der Kolonne angeordnet.

Zusätzlich kann die genannte Standregelung am Teilbereich 6, und damit für den Kolonnensumpf, als Stellgröße für die Seitenentnahmemenge genutzt werden. Hierzu wird als Regelgröße der Flüssigkeitsstand im Verdampfer verwendet.

Als Stellgröße der Heizleistung kann auch der Differenzdruck über die Kolonne genutzt werden. Günstigerweise wird die Destillation bei einem Druck zwischen 5 und 500 mbar, bevorzugt zwischen 10 und 200 mbar, durchgeführt. Der Druck wird dabei im Kopf der Kolonne gemessen. Dementsprechend wird zur Einhaltung dieses Druckbereiches die Heizleistung des Verdampfers V am Kolonnenboden gewählt.

Unter diesen Druckbedingungen liegt dann die Destillationstemperatur zwischen 50 und 200 °C, bevorzugt zwischen 80 und 150 °C. Die Destillationstemperatur ist dabei die Temperatur, die am Seitenabzug der Trennwandkolonne gemessen wird.

Somit ist das erfindungsgemäße Verfahren dadurch gekennzeichnet, dass der Kopfdruck in der Kolonne 5 bis 500 mbar beträgt.
Dann ist das erfindungsgemäße Verfahren auch dadurch gekennzeichnet, dass die Destillationstemperatur am Seitenabzug 50 bis 200 °C beträgt.

Um die Trennwandkolonne störungsfrei betreiben zu können, werden die genannten Regelmechanismen zumeist in Kombination angewendet.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelfraktion. Hierbei werden entweder einzelne für das Trennproblem kritische Komponenten, sogenannte Schlüsselkomponenten, oder die Summe von mehreren Schlüsselkomponenten spezifiziert.

Die Einhaltung der Spezifikation für die Hochsieder in der Mittelsiederfraktion wird vorzugsweise über das Aufteilungsverhältnis der Flüssigkeitsmenge am oberen Ende der Trennwand geregelt. Dabei wird das Aufteilungsverhältnis so eingestellt, dass die Konzentration an Schlüsselkomponenten für die Hochsiederfraktion in der Flüssigkeit am oberen Ende der Trennwand 10 bis 80 Gew.-%, bevorzugt 30 bis 50 Gew.-%, des Wertes ausmacht, der im Seitenabzug erzielt werden soll. Die Flüssigkeitsaufteilung kann dann so eingestellt werden, dass bei höheren Gehalten an Schlüsselkomponenten der Hochsiederfraktion mehr und bei niedrigeren Gehalten an Schlüsselkomponenten weniger Flüssigkeit auf den Zulaufteil geleitet wird.

Entsprechend wird die Spezifikation für die Leichtsieder in der Mittelsiederfraktion durch die Heizleistung geregelt. Hierbei wird die Heizleistung im Verdampfer V so eingestellt, dass die Konzentration an Schlüsselkomponenten für die Leichtsiederfraktion in der Flüssigkeit am unteren Ende der Trennwand 10 bis 80 Gew.-%, bevorzugt 30 bis 50 Gew.-%, des Wertes ausmacht, der im Seitenabzugsprodukt erzielt werden soll. Somit wird die Heizleistung dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Die Konzentration von Leicht- und Hochsiedern in der Mittelsiederfraktion kann nach üblichen Analysemethoden ermittelt werden. Beispielsweise kann zur Detektion Infrarot-Spektroskopie verwendet werden, wobei die im Reaktionsgemisch vorliegenden Verbindungen an Hand ihrer charakteristischen Absorptionen identifiziert werden. Diese Messungen können inline direkt in der Kolonne vorgenommen werden. Bevorzugt werden jedoch gaschromatographische Methoden verwendet. Hierbei ist dann vorgesehen, dass das obere und untere Ende der Trennwand Probeentnahmemöglichkeiten aufweist. Somit können aus der Kolonne kontinuierlich oder in zeitlichen Abständen flüssige oder gasförmige Proben entnommen und hinsichtlich ihrer Zusammensetzung untersucht werden. In Abhängigkeit von der Zusammensetzung kann dann auf die entsprechenden Regelmechanismen zurückgegriffen werden.

Es ist ein Ziel des erfindungsgemäßen Verfahrens, 1,2-Propylenglykol mit einer Reinheit von vorzugsweise mindestens 99 %, besonders bevorzugt 99,5 %, zur Verfügung zu stellen, wobei die Summe aus Wertstoff und den darin enthaltenen Schlüsselkomponenten 100 Gew.-% beträgt.

Im Wertstoff soll dann die Konzentration der Schlüsselkomponenten in den Leichtsiedern (z. B. Wasser, Methoxypropanol, Hydroxyaceton) und der Schlüsselkomponenten in den Hochsiedern (z. B. Di- und Tripropylenglykol) vorzugsweise unter 1 Gew.-%, besonders bevorzugt unter 0,5 Gew.-%, liegen.

In einer speziellen Ausführung der Trennwandkolonne ist es auch möglich, Zulaufteil und Entnahmeteil, die durch die Trennwand 7 voneinander separiert sind, nicht in einer Kolonne zu vereinen, sondern räumlich voneinander zu trennen. Somit kann in dieser speziellen Ausführung die Trennwandkolonne auch aus mindestens zwei voneinander räumlich getrennten Kolonnen bestehen, die dann aber miteinander thermisch gekoppelt sein müssen. Solche thermisch miteinander gekoppelten Kolonnen tauschen miteinander Dampf und Flüssigkeit aus, wobei die Energiezufuhr aber nur über eine Kolonne erfolgt. Diese spezielle Ausführung bietet den Vorteil, dass die thermisch miteinander gekoppelten Kolonnen auch unter verschiedenen Drücken betrieben werden können, wobei eine noch bessere Einstellung des zur Destillation erforderlichen Temperaturniveaus möglich sein kann als bei der herkömmlichen Trennwandkolonne.

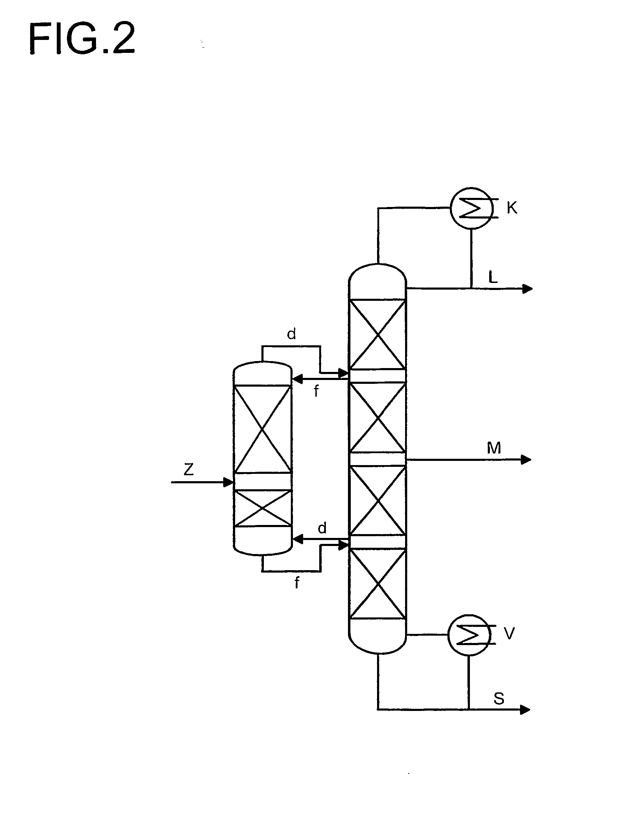

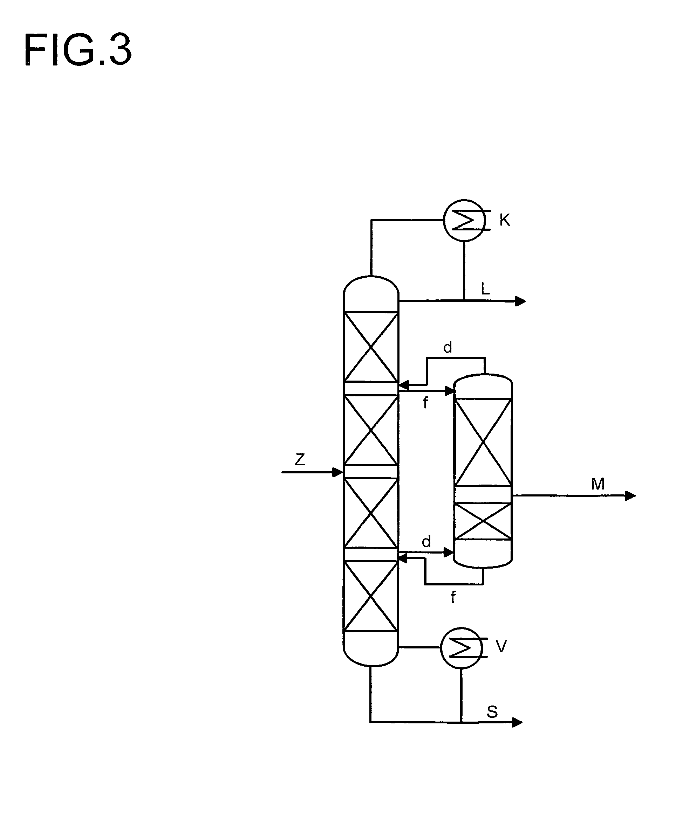

Beispiele für Trennwandkolonnen in der speziellen Ausführung der thermisch gekoppelten Kolonnen sind schematisch in Figur 2 und 3 dargestellt.

Figur 2 zeigt dabei eine Variante, in der die Energiezufuhr über den Verdampfer V der Kolonne erfolgt, die der Kolonne, über die der Abwasserstrom über den Zulauf Z eingespeist wird, nachgeschaltet ist. Dabei wird der Abwasserstrom in der ersten Kolonne zunächst in eine Leichtsieder- und eine Hochsiederfraktion, die auch Mittelsieder enthalten, aufgetrennt. Die resultierenden Fraktionen werden anschließend in die zweite Kolonne überführt, wobei die die Mittelsieder enthaltende Leichtsiederfraktion am oberen Ende und die die Mittelsieder enthaltende Hochsiederfraktion am unteren Ende der zweiten Kolonne eingespeist werden. Die Leichtsieder L werden über den Kopf der Kolonne abdestilliert und über den Kondensator K isoliert. Die Hochsieder S werden mit dem Sumpf der Kolonne erhalten. Aus dem Seitenabzug für Mittelsieder M kann das hochreine 1,2-Propylenglykol entnommen werden. Über d und f können beide Kolonnen Dampf und Flüssigkeit austauschen.

Figur 3 zeigt eine weitere Variante thermisch gekoppelter Kolonnen. In dieser Ausführungsform erfolgt die Energiezufuhr über den Verdampfer V der Kolonne, in die auch der Abwasserstrom über den Zulauf Z eingespeist wird. Über den Kopf dieser Kolonne werden die Leichtsieder L abdestilliert und mit Hilfe des Kondensators K kondensiert. Mit dem Sumpf werden die Hochsieder S erhalten. Mit Mittelsieder angereicherte Leichtsieder L werden nun in den oberen Teil der nachgeschalteten Kolonne überführt, mit Mittelsieder angereicherte Hochsieder S in den unteren Teil der nachgeschalteten Kolonne. Aus dem Seitenabzug für Mittelsieder M kann das hochreine 1,2-Propylenglykol entnommen werden. Über d und f können beide Kolonnen Dampf und Flüssigkeit austauschen.

Auch die Kolonnen nach Fig. 2 und 3 können als Packungskolonnen mit Füllkörpern oder geordneten Packungen oder als Bodenkolonnen ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Für das erfindungsgemäße Verfahren zur kontinuierlich betriebenen Reindestillation des bei der koppelproduktfreien Propylenoxidsynthese als Nebenprodukt entstehenden 1,2-Propylenglykols in einer Trennwandkolonne können für die Propylenoxidsynthese die aus dem Stand der Technik bekannten Hydroperoxide verwendet werden.

Beispiele für solche Hydroperoxide sind etwa tert-Butylhydroperoxid oder Ethylbenzolhydroperoxid. Bevorzugt wird als Hydroperoxid für die Propylenoxidsynthese Wasserstoffperoxid eingesetzt, wobei auch eine wässerige Wasserstoffperoxidlösung verwendet werden kann.

Zur Herstellung von Wasserstoffperoxid kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmann's Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Für die Propylenoxidsynthese aus dem Hydroperoxid und Propylen können zur größeren Effizienz der Umsetzung auch ein oder mehrere geeignete Katalysatoren zugesetzt werden, wobei wiederum heterogene Katalysatoren bevorzugt eingesetzt werden.

Es sind alle heterogenen Katalysatoren denkbar, die für die jeweilige Umsetzung geeignet sind. Bevorzugt werden dabei Katalysatoren verwendet, die ein poröses oxidisches Material, wie z. B. einen Zeolith, umfassen. Vorzugsweise werden Katalysatoren eingesetzt, die als poröses oxidisches Material einen Titan-, Germanium-, Tellur-, Vanadium-, Chrom-, Niob- oder Zirkonium-haltigen Zeolith umfassen.

Dabei sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind Ti-Zeolithe mit MFI-, MEL- oder MFI/MEL-Mischstruktur anzusehen. Ganz besonders bevorzugt sind im Einzelnen die Titan enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur.

Sehr günstig ist die Verwendung eines heterogenen Katalysator, der das titanhaltige Silikalit TS-1 umfasst.

Dabei ist es auch möglich, als Katalysator das poröse oxidische Material an sich zu verwenden. Selbstverständlich ist es jedoch auch möglich, als Katalysator einen Formkörper einzusetzen, der das poröse oxidische Material umfasst. Dabei können zur Herstellung des Formkörpers, ausgehend von dem porösen oxidischen Material, alle Verfahren gemäß dem Stand der Technik eingesetzt werden.

Vor, während oder nach dem einen oder mehreren Formgebungsschritten in diesen Verfahren können auf das Katalysatormaterial Edelmetalle in Form geeigneter Edelmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben.

Selbstverständlich können die Formkörper konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise vorstehend beschrieben.

Bei Verwendung eines Formkörpers oder auch mehr davon als Katalysator kann dieser im erfindungsgemäßen Verfahren nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Dieses Regenerierungsverfahren ist in der DE-A 197 23 949.8 beschrieben. Ferner können die dort bezüglich des Standes der Technik angegebenen Regenerierungsverfahren eingesetzt werden.

Als Lösungsmittel können alle Lösungsmittel verwendet werden, die die in die Propylenoxid-synthese eingesetzten Edukte ganz oder wenigstens teilweise lösen. Beispiele für Lösungsmittel sind aliphatische, cycloaliphatische und aromatische Kohlenwasserstoffe, Ester, Ether, Amide, Sulfoxide und Ketone sowie Alkohole. Die Lösungsmittel können dabei auch in Form von Mischungen eingesetzt werden. Bevorzugt werden Alkohole eingesetzt. Dabei ist der Einsatz von Methanol als Lösungsmittel besonders bevorzugt.

Im Allgemeinen liegt die Reaktionstemperatur für die Herstellung des Propylenoxids in den Stufen (i) und (iii) im Bereich von 0 bis 120 °C, bevorzugt im Bereich von 10 bis 100 °C und weiter bevorzugt im Bereich von 20 bis 90 °C. Die auftretenden Drücke reichen dabei von 1 bis 100 bar, vorzugsweise 1 bis 40 bar, mehr bevorzugt 1 bis 30 bar. Bevorzugt wird bei Drücken gearbeitet, unter denen keine Gasphase vorliegt.

Die Konzentration von Propylen und Wasserstoffperoxid im Eduktstrom wird im Allgemeinen so gewählt, dass das molare Verhältnis bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt.

Zur Reduktion der bei der Propylenoxidherstellung im Produktgemisch enthaltenen Hydroperoxyalkohole können die in der DE 101 05 527.7 beschriebenen Methoden herangezogen werden.

Beispielsweise ist es möglich, Phosphor(III)-Verbindungen, beispielsweise Phosphortrichlorid, Phosphane (z.B. Triphenylphosphin, Tributylphosphin), Phosphorige Säure bzw. deren Salze oder Natriumhypophosphit, einzusetzen.

Auch die reduktive Umsetzung mit Schwefel(II)-Verbindungen wie beispielsweise Schwefelwasserstoff oder Salze davon, Natriumpolysulfide, Dimethylsulfid, Tetrahydrothiophen, Bis-(hydroxyethyl)-sulfid oder Natriumthiosulfat sowie mit Schwefel(IV)-Verbindungen, wie beispielsweise Schweflige Säure und deren Salze, Natriumbisulfit oder Thioharnstoff-S-oxid, ist möglich.

Weitere Reduktionsmittel sind Nitrite, beispielsweise Natriumnitrit oder Isoamylnitrit. Auch α-Hydroxycarbonylverbindungen wie Hydroxyaceton, Dihydroxyaceton, 2-Hydroxycyclopentanon (Glutaroin), 2-Hydroxycyclohexanon (Adipoin), Glucose sowie andere reduzierbare Zucker sind geeignet. Endiole wie Ascorbinsäure oder Verbindungen, welche eine Bor-Wasserstoff-Bindung enthalten, beispielsweise Natriumborhydrid oder Natriumcyanborhydrid, sind gleichfalls verwendbar.

Bevorzugt werden zur Reduktion von α-Hydroperoxyalkohol-haltigen Produktgemischen jedoch katalytische Hydrierverfahren mit Wasserstoff gewählt, die in homogener wie auch heterogener Phase durchgeführt werden können. Der Hydrierkatalysator weist dabei mindestens ein Aktivmetall aus der VIIb-, der VIII-, der Ia- sowie der Ib-Nebengruppe des Periodensystems der Elemente, einzeln oder als Gemisch aus zwei oder mehr davon, auf. Beispielsweise können Palladium (Pd), Platin (Pt), Rhodium (Rh), Ruthenium (Ru), Iridium (Ir), Osmium (Os), Eisen (Fe), Cobalt (Co), Nickel (Ni) sowie Kupfer (Cu), bevorzugt Pd, Pt, Rh, Ru und Ir, besonders bevorzugt Pd eingesetzt werden. Diese Katalysatoren sind sowohl in Pulverform einsetzbar wie auch als Aktivmetallkörper. Dabei kommen bevorzugt Folien, Drähte, Netze, Granalien und Kristallitpulver, hergestellt aus mindestens einem Aktivmetall oder einer Mischung aus zwei oder mehr davon, zur Anwendung. Weiterhin sind auch Aktivmetalloxide, beispielsweise als Suspensionen mindestens eines Aktivmetalls oder einer Mischung aus zwei oder mehr davon, einsetzbar.

Als Reaktoren für die Propylenoxid-Synthese können selbstverständlich alle denkbaren, für die jeweiligen Reaktionen am besten geeigneten Reaktoren eingesetzt werden. Dabei ist für die Propylenoxid-Synthese ein Reaktor nicht auf einen einzelnen Behälter beschränkt. Vielmehr ist es auch möglich, beispielsweise eine Rührkesselkaskade einzusetzen.

Bevorzugt werden für die Propylenoxid-Synthese als Reaktoren Festbettreaktoren verwendet. Weiter bevorzugt werden als Festbettreaktoren Festbettrohrreaktoren eingesetzt.

Insbesondere wird für vorstehend beschriebene und bevorzugt verwendete Propylenoxid-synthese durch Umsetzung von Propylen mit einem Hydroperoxid, als Reaktor für die Stufe (i) ein isothermer Festbettreaktor und für die Stufe (üi) ein adiabatischer Festbettreaktor verwendet, wobei in Stufe (ii) das Hydroperoxid mittels einer Abtrennapparatur abgetrennt wird.

Somit fällt der für das erfindungsgemäße Verfahren eingesetzte Abwasserstrom, der das 1,2-Propylenglykol enthält, bei der Herstellung von Propylenoxid bevorzugt in einem isothermen Festbettreaktor und einem adiabatischen Festbettreaktor an.

Bei der Propylenoxidherstellung richten sich die Verweilzeiten im Reaktor bzw. in den Reaktoren dabei im Wesentlichen nach den gewünschten Umsätzen. Im Allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Auch ist es denkbar, zur Umsetzung mehrere Hydroperoxide zu verwenden. Werden Propylen und/oder mehrere Hydroperoxide miteinander in den jeweiligen Stufen umgesetzt, so können in den Mischungen verschiedenartige Produkte, die aus den Umsetzungen resultieren, vorliegen. Jedoch kann auch aus solchen Mischungen das 1,2-Propylenglykol mit gutem Erfolg nach dem erfindungsgemäßen Verfahren durch Reindestillation erhalten werden.

In einer bevorzugten Ausführung der Propylenoxidsynthese wird als Hydroperoxid Wasserstoffperoxid verwendet und Propylen während der Reaktion in Kontakt mit einem heterogenen Katalysator gebracht.

Weiterer Gegenstand der Erfindung ist auch eine Vorrichtung zur Durchführung eines kontinuierlich betriebenen Verfahrens zur Reindestillation des bei der Synthese von Propylenoxid als Nebenprodukt anfallenden 1,2-Propylenglykols, dadurch gekennzeichnet, dass die Vorrichtung wenigstens einen Reaktor zur Herstellung von Propylenoxid und wenigstens eine Trennwandkolonne zur Reindestillation des 1,2-Propylenglykols umfasst.

In einer speziellen Ausführung ist die Vorrichtung zur Durchführung eines kontinuierlich betriebenen Verfahrens zur Reindestillation des bei der Synthese von Propylenoxid als Nebenprodukt anfallenden 1,2-Propylenglykols dadurch gekennzeichnet, dass die Vorrichtung wenigstens einen isothermen und einen adiabatischen Reaktor für die Stufen (i) und (iii) sowie eine Abtrennapparatur für die Stufe (ii) zur Herstellung von Propylenoxid und wenigstens eine Trennwandkolonne zur Reindestillation des 1,2-Propylenglykols umfasst.

In einer speziellen Ausführung der Vorrichtung kann dabei die Trennwandkolonne auch aus mindestens zwei miteinander thermisch gekoppelten Kolonnen bestehen.

Die Erfindung soll nun durch folgendes Beispiel erläutert werden.

### Beispiel

Bei der koppelproduktfreien Herstellung von Propylenoxid aus Propylen und Wasserstoffperoxid nach dem in der WO 00/07965 beschriebenem Verfahren wurde ein Abwasserstrom erhalten, der 1,2-Propylenglykol enthielt und folgende Zusammensetzung aufwies:
ca. 10 Gew.-% leichtsiedende Komponenten umfassend die Schlüsselkomponenten Wasser, Methoxypropanole, Hydroxyaceton,
ca. 85 Gew.-% 1,2-Propylenglykol, und
ca. 5 Gew.-% hochsiedende Komponenten mit den Schlüsselkomponenten Di- und Tripropylenglykol.

Dieses Gemisch wurde mit Hilfe einer Trennwandkolonne destilliert, wobei das Gemisch der Kolonne kontinuierlich zugeführt wurde: Das 1,2-Propylenglykol wurde dabei als Mittelsieder dem Seitenabzug der Kolonne entnommen. Ziel der Destillation war es, die Summe der Verunreinigungen im 1,2-Propylenglykol durch Reindestillation auf unter 1 Gew.-% zu erniedrigen. Dazu wurde die Heizleistung der Sumpfverdampfer so geregelt, dass die Summe der Konzentrationen der Schlüsselkomponenten im Seitenabzug kleiner als 1 Gew.-% war.

Der benötigte Energieinhalt der Destillation wurde als Maß für die Effektivität der Trennung eingesetzt. Er wurde aus der Verdampferleistung berechnet bezogen auf den Stoffdurchsatz pro Stunde. Als Kolonnenverschaltungen wurden die in der Tabelle aufgeführten Anordnungen gewählt:

| Kolonnenverschaltung | Energiebedarf/(kg/h) [kW/(kg/h)] | Energieeinsparung [%] |
|---|---|---|
| Konventionelle Kolonne mit Seitenabzug | 0,99 | - |
| Reihenschaltung zweier konventioneller Kolonnen | 0,90 | 9,1 |
| Trennwandkolonne | 0,73 | 26,3 |

Es wird deutlich, dass die Trennwandverschaltung energetisch einen erheblichen Vorteil gegenüber den beiden konventionellen Destillationsanordnungen besaß, da der Energiebedarf für die Destillation des Wertstoffes im Vergleich zur Destillation in den konventionellen Destillationskolonnen deutlich niedriger lag.

### Bezugszeichenliste für Figuren 1, 2 und 3:

- 1: gemeinsamer Teilbereich des Zulauf- und Entnahmeteils der Trennwandkolonne
- 2: Verstärkungsteil des Zulaufteils
- 3: Abtriebsteil des Entnahmeteils
- 4: Abtriebsteil des Zulaufteils
- 5: Verstärkungsteil des Entnahmeteils
- 6: gemeinsamer Teilbereich des Zulauf- und Entnahmeteils
- 7: Trennwand

- Z: Zulauf
- L: Leichtsieder
- M: Seitenabzug für Mittelsieder
- S: Hochsieder

- K: Kondensator
- V: Verdampfer

- d: Dampf
- f: Flüssigkeit

Waagrechte und diagonale oder diagonal angedeutete Linien in den Kolonnen symbolisieren Packungen mit Füllkörpern oder geordnete Packungen, die in der Kolonne vorhanden sein können.

## Patentansprüche

1. Kontinuierlich betriebenes Verfahren zur Reindestillation des bei der Synthese von Propylenoxid als Nebenprodukt anfallenden 1,2-Propylenglykols, **dadurch gekennzeichnet, dass** das bei der Synthese entstehende Gemisch, das das 1,2-Propylenglykol enthält, in einer Trennwandkolonne in eine Leicht-, Mittel- und Hochsiederfraktion aufgetrennt und am Seitenabzug der Kolonne 1,2-Propylenglykol als Mittelsieder entnommen wird.

2. Verfahren nach Anspruch 1, **dadurch gekennzeichnet, dass** die Trennwandkolonne aus mindestens zwei thermisch gekoppelten Kolonnen besteht.

3. Verfahren nach Anspruch 1 oder 2, **dadurch gekennzeichnet, dass** die Trennwandkolonne 15 bis 60 theoretische Trennstufen besitzt.

4. Verfahren nach einem der Ansprüche 1 bis 3, **dadurch gekennzeichnet, dass** der Kopfdruck in der Trennwandkolonne 5 bis 500 mbar beträgt.

5. Verfahren nach einem der Ansprüche 1 bis 4, **dadurch gekennzeichnet, dass** die Destillationstemperatur am Seitenabzug der Trennwandkolonne 50 bis 200 °C beträgt.

6. Verfahren nach einem der Ansprüche 1 bis 5, **dadurch gekennzeichnet, dass** die Summe der Schlüsselkomponenten im gereinigten 1,2-Propylenglykol kleiner als 1 Gew.-% ist, wobei die Summe aus 1,2-Propylenglykol und Schlüsselkomponenten 100 Gew.-% beträgt.

7. Verfahren nach einem der Ansprüche 1 bis 6, **dadurch gekennzeichnet, dass** das das 1,2-Propylenglykol enthaltende Gemisch hergestellt wird durch ein Verfahren umfassend wenigstens die Schritte (i) bis (iii):
(i) Umsetzung des Hydroperoxids mit Propylen unter Erhalt eines Produktgemisches, umfassend Propylenoxid und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit Propylen.

8. Verfahren nach Anspruch 7, **dadurch gekennzeichnet, dass** in Stufe (i) ein isothermer Festbettreaktor und in Stufe (iii) ein adiabatischer Festbettreaktor sowie in Stufe (ii) eine Abtrennapparatur verwendet wird.

9. Verfahren nach einem der Ansprüche 1 bis 8, **dadurch gekennzeichnet, dass** als Hydroperoxid Wasserstoffperoxid verwendet und Propylen während der Reaktion in Kontakt mit einem heterogenen Katalysator gebracht wird.

10. Vorrichtung zur Durchführung eines kontinuierlich betriebenen Verfahrens zur Reindestillation des bei der Synthese von Propylenoxid mit Hydroperoxid und Propylen als Nebenprodukt anfallenden 1,2-Propylenglykols, **dadurch gekennzeichnet, dass** die Vorrichtung wenigstens einen isothermen und einen adiabatischen Festbettreaktor sowie eine Abtrennapparatur, in der nicht umgesetztes Hydroperoxid abgetrennt wird, und wenigstens eine Trennwandkolonne zur Reindestillation des 1,2-Propylenglykols umfasst, die mit einem Zulauf versehen ist, über den 1,2-Propylenglykol enthaltendes Abwasser aus der Propylenoxidsynthese in die Trennwandkolonne eingebracht wird, und wobei in dem isothermen Reaktor eine Umsetzung von Propylen mit Hydroperoxid unter Erhalt eines Produktgemisches, umfassend Propylenoxid und nicht umgesetztes Hydroperoxid, durchgeführt wird, und wobei in dem adiabatischen Reaktor das abgetrennte Hydroperoxid mit Propylen umgesetzt wird.

## Claims

1. A continuously operated process for the purification by distillation of the 1,2-propylene glycol formed as by-product in the synthesis of propylene oxide, wherein the mixture formed in the synthesis which contains the 1,2-propylene glycol is separated in a dividing wall column into low-, intermediate- and high-boiling fractions and the 1,2-propylene glycol is taken off as intermediate boiler at the side offtake of the column.

2. The process according to claim 1, wherein the dividing wall column comprises at least two thermally coupled columns.

3. The process according to claim 1 or 2, wherein the dividing wall column has from 15 to 60 theoretical plates.

4. The process according to any of claims 1 to 3, wherein the pressure at the top of the dividing wall column is from 5 to 500 mbar.

5. The process according to any of claims 1 to 4, wherein the distillation temperature at the side offtake of the dividing wall column is from 50 to 200°C.

6. The process according to any of claims 1 to 5, wherein the sum of the key components in the purified 1,2-propylene glycol is less than 1% by weight, with the sum of 1,2-propylene glycol and key components being 100% by weight.

7. The process according to any of claims 1 to 6, wherein the mixture containing 1,2-propylene glycol is prepared in a process comprising at least the steps (i) to (iii):
(i) reaction of the hydroperoxide with propylene to give a product mixture comprising propylene oxide and unreacted hydroperoxide,
(ii) separation of the unreacted hydroperoxide from the mixture resulting from step (i),
(iii) reaction of the hydroperoxide which has been separated off in step (ii) with propylene.

8. The process according to claim 7, wherein an isothermal fixed-bed reactor is used in step (i), an adiabatic fixed-bed reactor is used in step (iii) and a separation apparatus is used in step (ii).

9. The process according to any of claims 1 to 8, wherein the hydroperoxide used is hydrogen peroxide and propylene is brought into contact with a heterogeneous catalyst during the reaction.

10. An apparatus for carrying out a continuously operated process for the purification by distillation of the 1,2-propylene glycol formed as by-product in the synthesis of propylene oxide using hydroperoxide and propylene, which comprises at least one isothermal reactor and one adiabatic fixed-bed reactor and also a separation apparatus in which unreacted hydroperoxide is separated off, and at least one dividing wall column for purifying the 1,2-propylene glycol by distillation which is provided with a feed line via which wastewater comprising 1,2-propylene glycol from the propylene oxide synthesis is introduced into the dividing wall column, wherein a reaction of propylene with hydroperoxide to give a product mixture comprising propylene oxide and unreacted hydroperoxide is carried out in the isothermal reactor and the hydroperoxide which has been separated off is reacted with propylene in the adiabatic reactor.

## Revendications

1. Procédé opérant en mode continu pour la distillation à l'état pur de 1,2-propylèneglycol se formant comme sous-produit lors de la synthèse de l'oxyde de propylène, **caractérisé en ce que** le mélange se formant lors de la synthèse, qui contient le 1,2-propylèneglycol, est séparé dans une colonne à paroi séparatrice en une fraction à bas point d'ébullition, une fraction à point d'ébullition moyen et une fraction à haut point d'ébullition et **en ce que** l'on retire du 1,2-propylèneglycol sur le soutirage latéral de la colonne sous forme de substance à point d'ébullition moyen.

2. Procédé selon la revendication 1, **caractérisé en ce que** la colonne à paroi séparatrice est constituée d'au moins deux colonnes couplées thermiquement.

3. Procédé selon la revendication 1 ou 2, **caractérisé en ce que** la colonne à paroi séparatrice possède 15 à 60 étages de séparation théoriques.

4. Procédé selon l'une quelconque des revendications 1 à 3, **caractérisé en ce que** la pression de tête dans la colonne à paroi séparatrice est de 5 à 500 mbars.

5. Procédé selon l'une quelconque des revendications 1 à 4, **caractérisé en ce que** la température de distillation sur le soutirage latéral de la colonne à paroi séparatrice est de 50 à 200°C.

6. Procédé selon l'une quelconque des revendications 1 à 5, **caractérisé en ce que** la somme des composants clés dans le 1,2-propylèneglycol purifié est inférieure à 1% en poids, la somme du 1,2-propylèneglycol et des composants clés représentant 100% en poids.

7. Procédé selon l'une quelconque des revendications 1 à 6, **caractérisé en ce que** le mélange contenant le 1,2-propylèneglycol est fabriqué par un procédé comprenant au moins les étapes (i) à (iii) :
(i) réaction de l'hydroperoxyde avec du propylène pour obtenir un mélange de produits comprenant de l'oxyde de propylène et de l'hydroperoxyde qui n'a pas réagi,
(ii) séparation de l'hydroperoxyde qui n'a pas réagi du mélange obtenu à l'étape (i), et
(iii) réaction de l'hydroperoxyde séparé de l'étape (ii) avec du propylène.

8. Procédé selon la revendication 7, **caractérisé en ce que** l'on utilise à l'étape (i) un réacteur à lit fixe isothermique et à l'étape (iii) un réacteur à lit fixe adiabatique ainsi qu'à l'étape (ii) un appareillage de séparation.

9. Procédé selon l'une quelconque des revendications 1 à 8, **caractérisé en ce que** l'on utilise comme hydroperoxyde du peroxyde d'hydrogène et **en ce que** l'on met du propylène en contact avec un catalyseur hétérogène pendant la réaction.

10. Procédé pour réaliser un procédé opérant en mode continu pour la distillation à l'état pur du 1,2-propylèneglycol se formant comme produit secondaire lors de la synthèse d'oxyde de propylène avec de l'hydroperoxyde et du propylène, **caractérisé en ce que** le dispositif comprend au moins un réacteur à lit fixe isothermique et un réacteur à lit fixe adiabatique ainsi qu'un appareillage de séparation, dans lequel on sépare l'hydroperoxyde qui n'a pas réagi, et au moins une colonne à paroi séparatrice pour la distillation à l'état pur du 1,2-propylèneglycol qui est pourvue d'une alimentation par laquelle de l'eau usée contenant du 1,2-propylèneglycol et issue de la synthèse d'oxyde de propylène est introduite dans la colonne à paroi séparatrice, et dans lequel, dans le réacteur isothermique, une réaction de propylène avec de l'hydroperoxyde est effectuée pour obtenir un mélange de produits comprenant de l'oxyde de propylène et de l'hydroperoxyde qui n'a pas réagi, et dans lequel on fait réagir, dans le réacteur adiabatique, l'hydroperoxyde séparé avec du propylène.
